(19) Europäisches Patentamt
European Patent Office
Office européen des brevets

(11) **EP 3 600 199 B1**

(12) **EUROPEAN PATENT SPECIFICATION**

(45) Date of publication and mention
of the grant of the patent:
**18.05.2022   Bulletin 2022/20**

(21) Application number: **17901764.5**

(22) Date of filing: **21.03.2017**

(51) International Patent Classification (IPC):
**A61F 13/511** *(2006.01)*

(52) Cooperative Patent Classification (CPC):
**A61F 13/51113; A61F 13/4942; A61F 13/51104;
A61F 13/5116; A61F 13/51305; A61F 13/531;
A61F 13/537; A61F 13/539;** A61F 2013/15406;
A61F 2013/49093; A61F 2013/51117;
A61F 2013/530481; A61F 2013/5315;
A61F 2013/5395

(86) International application number:
**PCT/CN2017/077483**

(87) International publication number:
**WO 2018/170729 (27.09.2018 Gazette 2018/39)**

(54) **ABSORBENT ARTICLE HAVING A MULTI-COMPONENT TOPSHEET AND LEG CUFFS**

ABSORBIERENDER ARTIKEL MIT EINER MEHRKOMPONENTENDECKFOLIE UND
BEINMANSCHETTEN

ARTICLE ABSORBANT AYANT UNE FEUILLE SUPÉRIEURE MULTI-COMPOSANTS ET DES
REVERS DE JAMBE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**05.02.2020   Bulletin 2020/06**

(73) Proprietor: **The Procter & Gamble Company
Cincinnati, OH 45202 (US)**

(72) Inventors:
• **YUAN, Yi
Beijing 101312 (CN)**
• **BIANCHI, Ernesto Gabriel
65824 Schwalbach am Taunus (DE)**

• **ERDEM, Gueltekin
Beijing 101312 (CN)**

(74) Representative: **P&G Patent Germany
Procter & Gamble Service GmbH
Sulzbacher Straße 40
65824 Schwalbach am Taunus (DE)**

(56) References cited:
EP-A1- 1 842 513         WO-A1-99/07318
WO-A1-2012/043842    WO-A1-2014/085974
WO-A1-2015/094623    WO-A1-2015/134359
US-A1- 2015 250 658    US-A1- 2015 250 660
US-A1- 2017 056 256

**Description**

FIELD OF THE INVENTION

The present invention is generally related to an absorbent articles comprising multi-component topsheets and leg cuffs.

BACKGROUND OF THE INVENTION

Absorbent articles for personal hygiene, such as disposable diapers for infants, training pants for toddlers, adult incontinence undergarments, and/or sanitary napkins are designed to absorb and contain body exudates, in particular large quantities of urine, runny BM, and/or menses (together the "fluids"). These absorbent articles may comprise several layers providing different functions, for example, a topsheet, a backsheet, and an absorbent core disposed between the topsheet and the backsheet, and leg cuffs, among other layers (e.g., acquisition layer, distribution layer, etc.), if desired.

The topsheet is generally liquid permeable and is configured to receive the fluids being excreted from the body and aid in directing the fluids toward an acquisition system, a distribution system, and/or the absorbent core. In general, important qualities of a topsheet include a high liquid permeability and softness of the material and gentle skin feel. In addition, topsheets are expected to have short amount of time the fluids spending on topsheet prior to being absorbed by the absorbent article, so that the wearer feel dry and skin comfort during/after urination.

To improve desired qualities, materials for topsheets may be further processed. For example, one of approaches to solve the problem of the skin feeling wet because of prolonged fluid residency on the topsheets, topsheets is using three-dimensional substrates that further reduce skin/fluid contact and/or skin/fluid contact time during, for example, a urination event. As more favorable materials for a topsheet such as three-dimensional substrates materials can be relatively expensive when compared to traditional topsheet materials, a topsheet having a center topsheet of a more favorable substrate sandwiched between lateral topsheets of less expensive nonwoven substrates are suggested.

The presence of multiple-piece topsheet may not be favorable by customers in view of holistic product perception due to the presence of bonding areas where bondings and free edges of the overhang of the center topsheet or lateral topsheets. In addition, bondings joining the center topsheet and the lateral topsheets may cause less skin gentleness or less skin gentleness perception.

Therefore, there is continued interest to be able to attain the benefits of using substrates which handle fluid more efficient ways and reducing ponding of the fluids on the topsheet without compromising one-piece looking perception and skin gentleness, while limiting the added expense of employing such materials.

US 2015/250660 relates to a multi-component topsheet for an absorbent article includes a first discrete substrate, a second discrete substrate, and a third discrete substrate. The second discrete substrate is disposed at least partially intermediate the first discrete substrate and the third discrete substrate. The second discrete substrate is joined to the first discrete substrate and the third discrete substrate.

US 2017/056256 discloses an absorbent article comprises a three-dimensional liquid permeable topsheet. The topsheet comprises a first layer comprising a hydrophobic material and a second layer comprising a hydrophilic material. The first layer is joined to the second layer.

US 2015/250658 is concerned with a multi-component topsheet for an absorbent article and includes a first discrete substrate forming about 80% or more of an outer perimeter of the topsheet and a second discrete substrate. About 80% or more of an outer perimeter of the second discrete substrate is joined to the first discrete substrate. The topsheet has a single layer of substrate and a dual layer of substrate. The dual layer substrate is formed from an overlap between the first discrete substrate and the second discrete substrate.

SUMMARY OF THE INVENTION

The present invention is related to an absorbent article as defined in claim 1 and having a topsheet comprising a second substrate disposed at least partially intermediate a first discrete substrate and a third discrete substrate wherein the first discrete substrate opposing the third discrete substrate about a longitudinal axis of the topsheet, and wherein the first discrete substrate and the second discrete substrate are bonded in a first bonding zone about a longitudinal axis of the topsheet, and the third discrete substrate and the second discrete substrate are bonded in a second bonding zone about a longitudinal axis of the topsheet; and a pair of leg cuffs having free terminal edges and being joined to the topsheet and/or backsheet, wherein the distance between the free terminal edges is not longer than the distance between an inner edge of the first bonding zone and an inner edge of the second bonding zone.

The present invention is also related to an absorbent article as defined in claim 16 and having a topsheet comprising a first discrete substrate forming about 80% or more of an outer perimeter of the topsheet, and a second discrete substrate wherein about 80% or more of an outer perimeter of the second discrete substrate is joined to the first discrete substrate; and a pair of leg cuffs, each of the leg cuffs having a free terminal edge and being joined to the topsheet and/or backsheet, wherein the distance between the free terminal edges is not longer than the distance between an inner edge of the first bonding zone and an inner edge of the second bonding zone.

BRIEF DESCRIPTION OF THE DRAWINGS

The above-mentioned and other features and advantages of the present disclosure, and the manner of attaining them, will become more apparent and the disclosure itself will be better understood by reference to the following description of non-limiting forms of the disclosure taken in conjunction with the accompanying drawings, wherein:

Fig. 1 is a top view of an absorbent article, wearer-facing surface facing the viewer, with some layers partially removed in accordance with the present disclosure;
Fig. 2 is a cross-sectional view of the absorbent article taken about line 2-2 of Fig. 1 in accordance with the present disclosure;
Fig. 3 is a schematic illustration of a top view of a multi-component topsheet and leg cuffs in accordance with the present disclosure;
Fig. 4 is a schematic illustration of a cross-sectional view of Fig. 3;
Fig. 5 is a schematic illustration of a cross-sectional view of Fig. 3;
Fig. 6 is a schematic illustration of a top view of a multi-component topsheet and leg cuffs in accordance with the present disclosure;
Fig. 7 is a schematic illustration of a cross-sectional view of Fig. 6;
Fig. 8 is a schematic illustration of a top view of a multi-component topsheet and leg cuffs in accordance with the present disclosure;
Fig. 9 is a schematic illustration of a cross-sectional view of Fig. 8;
Fig. 10 is a schematic illustration of a cross-sectional view of Fig. 8;
Fig. 11 is a schematic illustration of a top view of a multi-component topsheet and leg cuffs in accordance with the present disclosure;
Fig. 12 is a top view of an absorbent article, wearer-facing surface facing the viewer, that comprises a multi-component topsheet and leg cuffs in accordance with the present disclosure; and
Fig. 13A is an enlarged top view of a portion of the absorbent article of Fig. 12 in accordance with the present disclosure.
Fig. 13B is an enlarged top view of a portion of the absorbent article of Fig. 12 in accordance with the present disclosure.

DETAILED DESCRIPTION

Various non-limiting forms of the present disclosure will now be described to provide an overall understanding of the principles of the structure, function, manufacture, and use of an absorbent article having a multi-component topsheet and leg cuffs disclosed herein. One or more examples of these non-limiting embodiments are illustrated in the accompanying drawings. Those of ordinary skilled in the art will understand that the absorbent articles described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

As used herein, the term "absorbent article" refers to disposable devices such as infant, child, or adult diapers, adult incontinence products, training pants, sanitary napkins and the like which are placed against or in proximity to a body of a wearer to absorb and contain the various fluids (urine, menses, and/or runny BM) or bodily exudates (generally solid BM) discharged from the body. Typically, these absorbent articles comprise a topsheet, backsheet, an absorbent core, leg cuffs, optionally an acquisition system and/or a distribution system (which may be comprised of one or several layers), and typically other components, with the absorbent core normally placed at least partially between the backsheet and the acquisition and/or distribution system or between the topsheet and the backsheet. The absorbent articles comprising a multi-component topsheet and leg cuffs of the present disclosure will be further illustrated in the below description and in the Figures in the form of one or more components of taped diaper. Nothing in this description should be, however, considered limiting the scope of the claims. As such the present disclosure applies to any suitable form of absorbent articles (e.g., diapers, training pants, adult incontinence products, sanitary napkins).

As used herein, the term "nonwoven web" means a manufactured sheet, web, or batt of directionally or randomly oriented fibers, bonded by friction, and/or cohesion, and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers may have diameters ranging from less than about 0.001 mm to more than about 0.2 mm and may come in several different forms such as short fibers (known as staple, or chopped), continuous single fibers (filaments or monofilaments), untwisted bundles of continuous filaments (tow), and twisted bundles of continuous filaments (yam). Nonwoven webs may be formed by many processes such as meltblowing, spunbonding, solvent spinning, electro spinning, carding, and airlaying. The basis weight of nonwoven webs is usually expressed in grams per square meter (g/m$^2$ or gsm).

As used herein, the terms "joined", "bonded", or "attached" encompasses configurations whereby an element is directly secured to another element by affixing the element directly to the other element, and configurations whereby an element is indirectly secured to another element by affixing the element to intermediate member(s) which in turn are affixed to the other element.

As used herein, the term "machine direction" or "MD" is the direction that is substantially parallel to the direction of travel of a substrate as it is made. The "cross direction" or "CD" is the direction substantially perpendicular to the MD and in the plane generally defined by the substrate.

General Description of the Absorbent Article

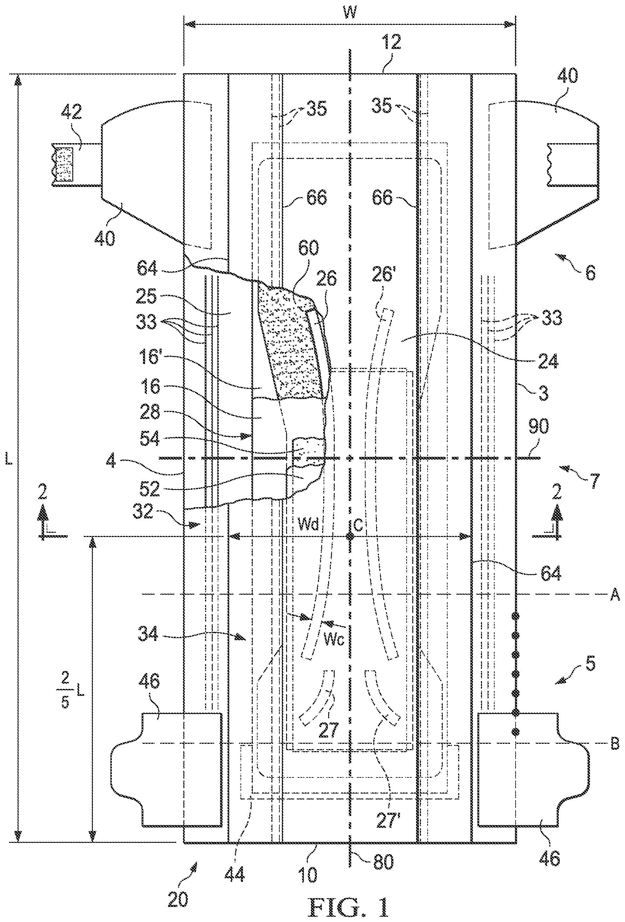

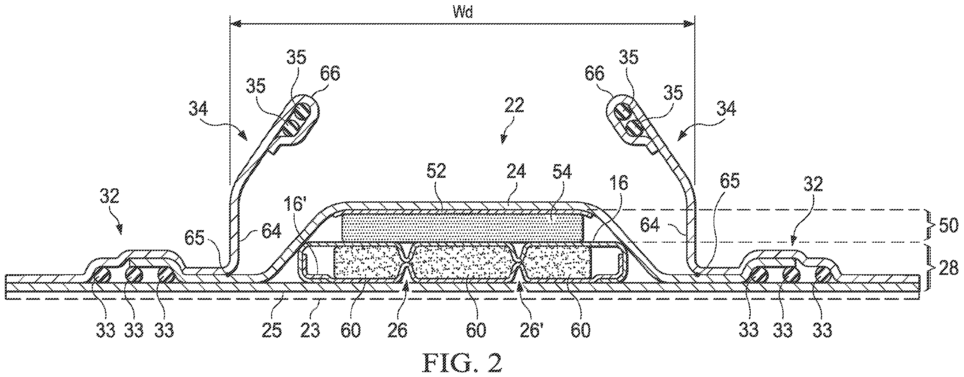

An example absorbent article in the form of diaper 20 is represented in Figs. 1 and 2. Fig. 1 is a plan view of the example diaper 20, in a flat-out state, with portions of the structure being cut-away to more clearly show the construction of the diaper 20. The wearer-facing surface of the diaper 20 of Fig. 1 is facing the viewer. This diaper 20 is shown for illustration purpose only and the absorbent article of the present invention can be any of various types of absorbent articles.

The absorbent article 20 comprises a liquid permeable topsheet 24, a liquid impermeable backsheet 25, an absorbent core 28 positioned at least partially intermediate the topsheet 24 and the backsheet 25, and leg cuffs 34. The absorbent article may also comprise an acquisition and/or distribution system ("ADS") 50, which in the example represented comprises a distribution layer 54 and an acquisition layer 52, which will be further detailed below. The absorbent article may also comprise elasticized gasketing cuffs 32 comprising elastics 33 joined to a chassis of the absorbent article, typically via the topsheet and/or backsheet, and substantially planar with the chassis of the diaper.

The figures also show typical taped diaper components such as a fastening system comprising tabs 42 attached towards the rear edge of the article and cooperating with a landing zone 44 on the front of the absorbent article. The absorbent article may also comprise other typical elements, which are not represented, such as a rear elastic waist feature, a front elastic waist feature, transverse barrier cuff(s), and/or a lotion application, for example.

The absorbent article 20 comprises a front waist edge 10, a rear waist edge 12 longitudinally opposing the front waist edge 10, a first side edge 3, and a second side edge 4 laterally opposing the first side edge 3. The front waist edge 10 is the edge of the article which is intended to be placed towards the front of the user when worn, and the rear waist edge 12 is the opposite edge. The absorbent article may have a longitudinal axis 80 extending from the lateral midpoint of the front waist edge 10 to a lateral midpoint of the rear waist edge 12 of the article and dividing the article in two substantially symmetrical halves relative to the longitudinal axis 80, with the article placed flat and viewed from above as in Fig. 1. The absorbent article may also have a lateral axis 90 extending from the longitudinal midpoint of the first side edge 3 to the longitudinal midpoint of the second side edge 4. The length, L, of the article may be measured along the longitudinal axis 80 from the front waist edge 10 to the rear waist edge 12. The width, W, of the article may be measured along the lateral axis 90 from the first side edge 3 to the second side edge 4. The article may comprise a crotch point C defined herein as the point placed on the longitudinal axis at a distance of two fifth (2/5) of L starting from the front edge 10 of the article 20. The article may comprise a front waist region 5, a rear waist region 6, and a crotch region 7. The front waist region 5, the rear waist region 6, and the crotch region 7 each define 1/3 of the longitudinal length, L, of the absorbent article.

The topsheet 24, the backsheet 25, the absorbent core 28, and the other article components may be assembled in a variety of configurations, in particular by gluing, heat embossing or ultrasonic bonding known in absorbent article manufacturing industry.

The absorbent core 28 may comprise an absorbent material comprising superabsorbent polymers and a core wrap enclosing the superabsorbent polymers. The core wrap may typically comprise two materials, substrates, or nonwoven materials 16 and 16' for the top side and bottom side of the core. The core may comprises one or more channels, represented in Fig. 1 as the four channels 26, 26' and 27, 27'. The channels 26, 26', 27, and 27' are optional features. Instead, the core may not have any channels or may have any number of channels.

These and other components of the example absorbent article will now be discussed in more details.

Topsheet

The topsheet 24 may be the part of the absorbent article that is in contact with the wearer's skin and receives the fluids. The topsheet 24 may be joined to the backsheet 25, the core 28 and/or any other layers as is known to those of skill in the art. Usually, the topsheet 24 and the backsheet 25 are joined directly to each other in some locations (e.g., on or close to the periphery of the absorbent article) and are indirectly joined together in other locations by directly joining them to one or more other elements of the article 20.

The topsheet 24 may be compliant, soft-feeling, and non-irritating to the wearer's skin. Further, a portion of, or all of, the topsheet 24 may be liquid permeable, permitting liquids to readily penetrate through its thickness. A suitable topsheet may be manufactured from a wide range of materials, such as porous foams, reticulated foams, apertured plastic films, or woven or nonwoven materials of natural fibers (e.g., wood or cotton fibers), synthetic fibers or filaments, or a combination of natural and synthetic fibers.

Any portion of the topsheet 24 may be coated with a lotion, a skin care composition, and/or antibacterial agents as is generally disclosed in the art. Further, the topsheet 24, the backsheet 25 or any portion of the topsheet or backsheet may be embossed and/or matte finished to provide a more cloth like appearance.

Backsheet

The backsheet 25 is generally that portion of the absorbent article 20 positioned adjacent the garment-facing surface of the absorbent core 28 and which prevents, or at least inhibits, the fluids and bodily exudates absorbed and contained therein from soiling articles such as bedsheets and undergarments. The backsheet 25 is typically impermeable, or at least substantially impermeable, to fluids (e.g., urine). The backsheet may, for example, be or comprise a thin plastic film such as a thermoplastic film having a thickness of about 0.012 mm to about 0.051 mm. Other suitable backsheet materials may include breathable materials which permit vapors to escape from the absorbent article 20 while still preventing, or at least inhibiting, fluids from passing through the backsheet 25. Example breathable materials may include materials such as woven webs, nonwoven webs, composite materials such as film-coated nonwoven webs, microporous films.

The backsheet 25 may be joined to the topsheet 24, the absorbent core 28, and/or any other element of the absorbent article 20 by any attachment methods known to those of skill in the art. Suitable attachment methods are described above with respect to methods for joining the topsheet 24 to other elements of the article 20.

An outer cover 23 may cover at least a portion of, or all of, the backsheet 25 to form a soft garment-facing surface of the absorbent article. The outer cover 23 may be formed of one or more nonwoven materials. The outer cover 23 may be joined to at least a portion of the backsheet 25 through mechanical bonding, adhesive bonding, or other suitable methods of attachment.

Absorbent Core

As used herein, the term "absorbent core" refers to the component of the absorbent article having the most absorbent capacity and comprising an absorbent material and a core wrap or core bag enclosing the absorbent material. The term "absorbent core" does not include the acquisition and/or distribution system or any other components of the article which are not either integral part of the core wrap or core bag or placed within the core wrap or core bag. The absorbent core may comprise, consist essentially of, or consist of, a core wrap, an absorbent material (e.g., superabsorbent polymers) as discussed, and glue.

The absorbent core 28 may comprise an absorbent material with a high amount of superabsorbent polymers (herein abbreviated as "SAP") enclosed within the core wrap. The SAP content may represent 70%-100% or at least 70%, 75%, 80%, 85%, 90%, 95%, 99%, or 100%, by weight of the absorbent material, contained in the core wrap. The core wrap is not considered as absorbent material for the purpose of assessing the percentage of SAP in the absorbent core. The core may also contain airfelt or cellulosic fibers with or without SAP.

By "absorbent material" it is meant a material which has some absorbency property or liquid retaining properties, such as SAP, cellulosic fibers as well as synthetic fibers. Typically, glues used in making absorbent cores have no or little absorbency properties and are not considered as absorbent material. The SAP content may be higher than 80%, for example at least 85%, at least 90%, at least 95%, at least 99%, and even up to and including 100% of the weight of the absorbent material contained within the core wrap. This provides a relatively thin core compared to a conventional core typically comprising between 40-60% SAP and high content of cellulose fibers. The conventional cores are also within the scope of the present disclosure. The absorbent material may in particular comprises less than 15% weight percent or less than 10% weight percent of natural, cellulosic, or synthetic fibers, less than 5% weight percent, less than 3% weight percent, less than 2% weight percent, less than 1% weight percent, or may even be substantially free of natural, cellulosic, and/or synthetic fibers.

Referring to Figs. 1 and 2, the core 28 may have a longitudinal axis corresponding substantially to the longitudinal axis 80 of the absorbent article 20. The absorbent material may be distributed in higher amount towards the front side than towards the rear side as more absorbency may be required at the front in particular absorbent articles. The core wrap may be formed by two nonwoven materials, laminates, or other materials, 16, 16' which may be at least partially sealed along two longitudinal sides of the absorbent core 28. The core wrap may be at least partially sealed along its front side and rear side, and two longitudinal sides so that substantially no absorbent material leaks out of the absorbent core wrap. The first material 16 may at least partially surround the second material 16' to form the core wrap. The first material 16 may surround a portion of the second material 16' proximate to the first and second longitudinal sides.

The absorbent core 28 may comprise adhesive, for example, to help immobilizing the SAP within the core wrap and/or to ensure integrity of the core wrap, in particular when the core wrap is made of two or more substrates. The adhesive may be a hot melt adhesive, supplied, by H.B. Fuller, for example. The core wrap may extend to a larger area than strictly needed for containing the absorbent material within.

The absorbent material may be a continuous layer present within the core wrap. Alternatively, the absorbent material may be comprised of individual pockets or stripes of absorbent material enclosed within the core wrap. In the first case, the absorbent material may be, for example, obtained by the application of a single continuous layer of absorbent material. The absorbent core 28 may also comprise a fibrous thermoplastic adhesive material at least partially bonding a layer of absorbent material 60 to material 16 or 16'. The thermoplastic adhesive used for the fibrous layer may have elastomeric properties, such that the web formed by the fibers on the SAP layer is able to be stretched as the SAP swell.

An absorbent material deposition area which may be defined by the periphery of the layer formed by the absorbent material 60 within the core wrap, as seen from the top side of the absorbent core may have various shapes, in particular, a so-called "dog bone" or "hour-glass" shape, which shows a tapering along its width towards the middle or "crotch" region of the core. In this way, the absorbent material deposition area may have a relatively narrow width in an area of the core intended to be placed in the crotch region of the absorbent article, as illustrated in Fig. 1. The absorbent material deposition area may comprise at least one channel 26, which is at least partially oriented in the longitudinal direction of the article 80 (i.e., has a longitudinal vector component). Leg Cuffs

The absorbent article comprises a pair of leg cuffs 34. Each barrier leg cuff may be formed by a piece of material which is bonded to the article so it may extend upwards from a wearer-facing surface of the absorbent article and provide improved containment of fluids and other body exudates approximately at the junction of the torso and legs of the wearer. The leg cuffs are delimited by a proximal edge 64 joined directly or indirectly to the topsheet 24 and/or the backsheet 25 and a free terminal edge 66, which is intended to contact and form a seal with the wearer's skin. The leg cuffs 34 extend at least partially between the front waist edge 10 and the rear waist edge 12 of the absorbent article on opposite sides of the longitudinal axis 80 and are at least present at the level of the crotch point (C) or crotch region. The leg cuffs may be joined at the proximal edge 64 with the chassis of the article by a cuff bond 65 which may be made by gluing, fusion bonding, or a combination of other suitable bonding processes. The cuff bond 65 at the proximal edge 64 may be continuous or intermittent. The cuff bond 65 closest to the raised section of the leg cuffs delimits the proximal edge 64 of the standing up section of the leg cuffs.

The leg cuffs may be integral with the topsheet 24 or the backsheet 25 or may be a separate material joined to the article's chassis. Each leg cuff 34 may comprise one, two or more elastic strings 35 close to the free terminal edge 66 to provide a better seal.

In addition to the leg cuffs 34, the article may comprise gasketing cuffs 32, which are joined to the chassis of the absorbent article, in particular to the topsheet 24 and/or the backsheet 25 and are placed externally relative to the leg cuffs. The gasketing cuffs 32 may provide a better seal around the thighs of the wearer. Each gasketing leg cuff may comprise one or more elastic strings or elastic elements 33 in the chassis of the absorbent article between the topsheet 24 and backsheet 25 in the area of the leg openings. All, or a portion of, the leg cuffs and/or gasketing cuffs may be treated with a lotion or another skin care composition.

Acquisition-Distribution System

The absorbent articles of the present disclosure may comprise an acquisition-distribution layer or system 50 ("ADS"). One function of the ADS is to quickly acquire one or more of the fluids and distribute them to the absorbent core in an efficient manner. The ADS may comprise one, two or more layers, which may form a unitary layer or may remain as discrete layers which may be attached to each other. In an example, the ADS may comprise two layers: a distribution layer 54 and an acquisition layer 52 disposed between the absorbent core and the topsheet, but the present disclosure is not so limited. The ADS may comprise SAP as this may slow the acquisition and distribution of the fluids.

The ADS 50 may comprise a distribution layer 54. The distribution layer of the ADS may comprise at least 50% by weight of cross-linked cellulose fibers. The cross-linked cellulosic fibers may be crimped, twisted, or curled, or a combination thereof including crimped, twisted, and curled. This type of material is disclosed in U.S. Pat. Publ. No. 2008/0312622 A1 (Hundorf).

The ADS 50 may comprise an acquisition layer 52. The acquisition layer may be disposed between the distribution layer 54 and the topsheet 24. The acquisition layer 52 may be or may comprise a nonwoven material, such as a hydrophilic SMS or SMMS material, comprising a spunbonded, a melt-blown and a further spunbonded layer or alternatively a carded staple fiber chemical-bonded nonwoven. The nonwoven material may be latex bonded.

In one example, the ADS may not be provided, or only one layer of the ADS may be provided, such as the distribution layer only or the acquisition layer only.

Fastening System

The absorbent article may include a fastening system. The fastening system may be used to provide lateral tensions about the circumference of the absorbent article to hold the absorbent article on the wearer as is typical for taped diapers. The fastening system may comprise a fastener such as tape tabs, hook and loop fastening components, interlocking fasteners such as tabs & slots, buckles, buttons, snaps, and/or hermaphroditic fastening components, although any other suitable fastening mechanisms are also within the scope of the present disclosure. A landing zone 44 is normally provided on the garment-facing surface of the front waist region 5 for the fastener to be releasably attached thereto.

Front and Rear Ears

The absorbent article may comprise front ears 46 and rear ears 40. The ears may be an integral part of the chassis, such as formed from the topsheet 24 and/or backsheet 26 as side panels. Alternatively, as represented on Fig. 1, the ears may be separate elements attached by gluing, heat embossing, and/or pressure bonding. The rear ears 40 may be stretchable to facilitate the attachment of the tabs 42 to the landing zone 44 and maintain the taped diapers in place around the wearer's waist. The rear ears 40 may also be elastic or extensible to provide a more comfortable and contouring fit by initially conformably fitting the absorbent article to the wearer and sustaining this fit throughout the time of wear well past when absorbent article has been loaded with fluids or other bodily exudates since the elasticized ears allow the sides of the absorbent article to expand and contract.

Multi-Component Topsheet

The absorbent article according to the present invention comprises a multi-component topsheet which comprises a first discrete substrate and a second discrete substrate which is joined to the first discrete substrate. The second discrete substrate may locate to across at least part of the longitudinal axis and a lateral axis of the absorbent article. The multi-component topsheet in the present invention has a dual layer substrate and a single layer substrate when an area of the multi-component topsheet where two discrete substrates are overlapped is defined as a dual layer of substrate, and an area of the multi-component that is not the dual layer of substrate is defined as a single layer substrate.

In one embodiment, the multi-component topsheet in the present invention comprises a first discrete substrate having an inner lateral edge in a longitudinal direction, a second discrete substrate, and a third discrete substrate having an inner lateral edge in a longitudinal direction, wherein the second discrete substrate is disposed at least partially intermediate the first discrete substrate and the third discrete substrate, wherein the first discrete substrate opposes the third discrete substrate about the longitudinal axis of the topsheet.

In another embodiment, the multi-component topsheet comprises a first discrete substrate forming about 80% or more of an outer perimeter of the topsheet and a second discrete substrate wherein about 80% or more of an outer perimeter of the second discrete substrate is joined to the first discrete substrate, wherein the topsheet has a single layer of substrate in about 75% or more of the total area of the topsheet and a dual layer of substrate in about 25% or less of the total area of the topsheet.

In the multi-component topsheet in the present invention, the second discrete substrate may be a three-dimensional, liquid permeable substrate. The three-dimensional, liquid permeable substrates of the present disclosure may comprise a substrate comprising an element selected from the group consisting of a plurality of projections, a plurality of recesses, a plurality of apertures and combinations thereof.

As one example, a three-dimensional substrate may comprises a plurality of projections that have a first z-directional height and land areas that have a second z-directional height, and optionally a plurality of apertures. The substrates may also have at least third elements having at least a third z-directional height as disclosed in WO2015/134371. Owing to such structures, fluids may be quickly moved away from the skin of a wearer, leaving primarily the first elements having the first z-directional heights contacting the skin of the wearer, thereby making the wearer feel dryer. The fluids may flow via gravity or via capillary gradient into the second elements having the second z-directional heights and/or into and through the apertures, so that the fluids may be absorbed into the absorbent articles.

The multi-component topsheet and absorbent articles comprising the multi-component topsheet of the present disclosure may be made by any suitable methods known in the art.

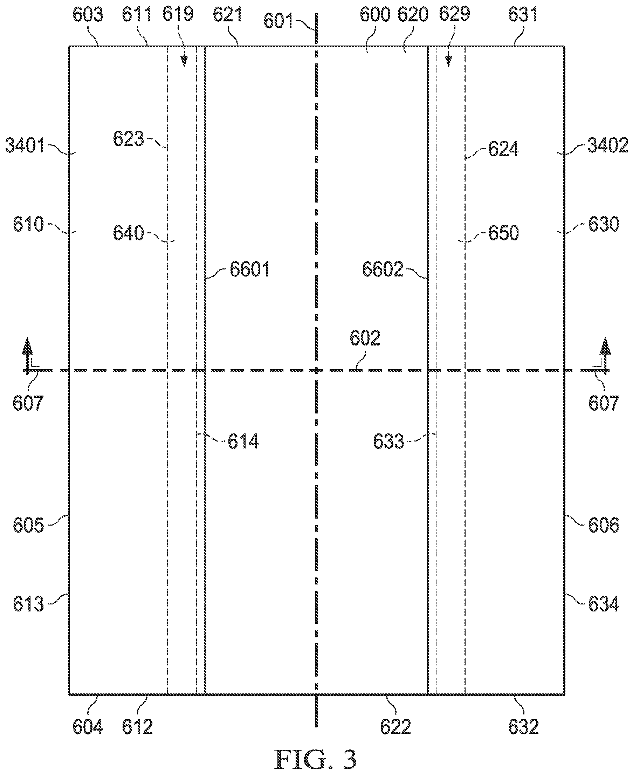

Figs. 3-5 depict top and cross-sectional schematic illustrations of embodiments of an absorbent article of the present invention having the multi-component topsheet 600 and leg cuffs 3401 and 3402. For the purpose of convenience of illustrations and descriptions of the present invention, only a multi-component topsheet and part of leg cuffs are illustrated, and other elements and/or layers of absorbent articles are omitted. The multi-component topsheet 600 may include a first discrete substrate 610, a second discrete substrate 620, and a third discrete substrate 630. The multi-component topsheet 600 has a longitudinal axis 601 that runs the longer overall dimension of the topsheet, and a lateral axis 602 that runs perpendicular to the longitudinal axis. The multi-component topsheet 600 may have an overall outer perimeter defined by first longitudinal edge 603, second longitudinal edge 604, first lateral edge 605, and second lateral edge 606. The first discrete substrate may have a perimeter defined by first longitudinal edge 611, second longitudinal edge 612, outer lateral edge 613, and inner lateral edge 614. The second discrete substrate may have a perimeter defined by first longitudinal edge 621, second longitudinal edge 622, first lateral edge 623, and second lateral edge 624. The third discrete substrate may have a perimeter defined by first longitudinal edge 631, second longitudinal edge 632, inner lateral edge 633, and outer lateral edge 634. Though Figs. 3-5 illustrate the lateral edges of leg cuffs 3401 and 3402 overlap the first and second lateral edge 605 and 606 of the topsheet 600, respectively, it does not mean the lateral edges of leg cuffs 3401 and 3402 should overlap the first and second lateral edge 605 and 606 of the topsheet 600, respectively. The lateral edge of a leg cuff may extend outwardly the lateral edges of the topsheet.

As shown in the embodiments of Figs. 3-5, the second discrete substrate 620 may be disposed at least partially intermediate the first discrete substrate 610 and the third discrete substrate 630 along a longitudinal direction (running the same directional as the longitudinal axis 601).

In some embodiments, the first and/or second longitudinal edges of the first discrete substrate 610, the second discrete substrate 620, and/or the third discrete substrate 630 will be common with the longitudinal edges 603, 604 of the multi-component topsheet 600.

In some embodiments, the outer lateral edge of the first discrete substrate 610 and/or the first lateral edge of the second discrete substrate 620, and the outer lateral edge of the third discrete substrate 630 and/or the second lateral edge of the second discrete substrate 620 will be common with the lateral edges 605, 606 of the multi-component topsheet 600, respectively.

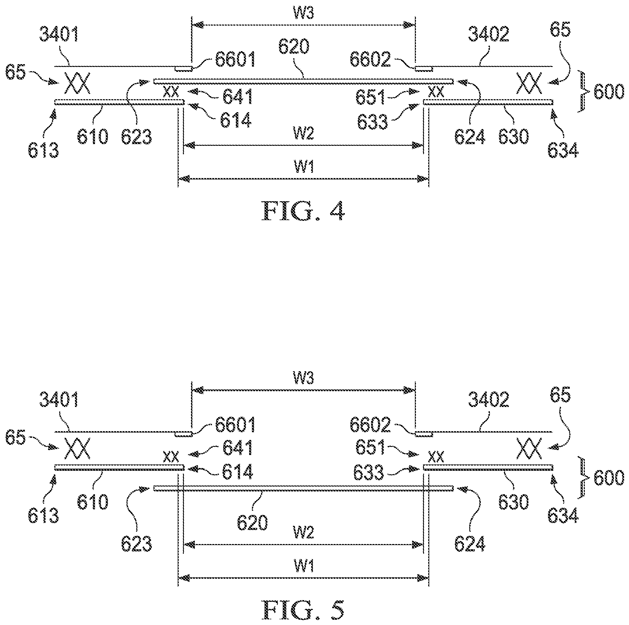

Fig. 3 schematically illustrates a top view of a body facing side of embodiments of an absorbent article of the present invention having a multi-component topsheet 600 and leg cuffs 3401, 3402 detailed herein. Figs. 4 and 5 depict cross sectional views of Fig. 3 in different embodiments, taken about line 607. As shown in Figs. 3-5, the second substrate 620 is disposed at least partially intermediate the first discrete substrate 610 and the third discrete substrate 630 along a longitudinal direction. The second substrate 620 is joined to the first discrete substrate 610 in a first bonding zone 619 and to the third discrete substrate 630 in a second bonding zone 629. The first and second bonding zones have inner lateral edges close to the inner lateral edge 614 of the first discrete substrate 610 and the inner lateral edge 633 of the third discrete substrate 630, respectively. When the bonding zone 619, 629 has a plurality of bondings, an inner lateral edge of the bonding zone means an inner lateral edge of a bonding which is closest to the inner lateral edge of the first or third discrete substrate. The bonding zone 619, 629 means an area where two discrete substrates are joined together. Widths of the first bonding zone 619 and the second bonding zone 629 may be the same as or shorter to width of a first dual layer area 640 and a second dual layer area 650 explained below, respectively. The joining of discrete substrates may be made by any method known in the art, including, but not limited to, mechanical bonding, hydroentangling, embossing, adhesive bonding, pressure bonding, heat bonding, ultrasonic bonding or by other methods of joining multiple discrete substrates. The non-limiting embodiments of Figs. 3-5 show the substrates joined by mechanical bonding 641, 651.

The overlap between the first discrete substrate 610 and the second discrete substrate 620 is, from a top view, the area contained by a perimeter consisting of the first lateral edge 623 of the second discrete substrate 620, the inner lateral edge 614 of the first discrete substrate 610, the first longitudinal edge 621 of the second discrete substrate 620, and the second longitudinal edge 622 of the second discrete substrate 620. Because this area contains two layers of substrate, it is referred to as the first dual layer area 640. The overlap between the second discrete substrate 620 and the third discrete substrate 630 is, from a top view, the area contained by a perimeter consisting of the second lateral edge 624 of the second discrete substrate 620, the inner lateral edge 633 of the third discrete substrate 630, the first longitudinal edge 621 of the second discrete substrate 620, and the second longitudinal edge 622 of the second discrete substrate 620, and is referred to as the second dual layer area 650. The first dual layer area 640 and the second dual layer area 650 add up to form the dual layer of substrate of the multi-component topsheet 600.

Widths in a lateral direction of the first and second dual layer area may be the same. Widths in a lateral direction of the first and second dual layer areas 640, 650 may be the same. Referring to the non-limiting embodiments depicted in Figs. 3-5, the width in a lateral direction of the first discrete substrate, i.e., the distance between the outer lateral edge 613 and the inner lateral edge 614 of the first discrete substrate 610 may be between about 20mm and about 70mm. The distance between the outer lateral edge 613 of the first discrete substrate 610 and the first lateral edge 623 of the second discrete substrate 620 may be between about 16mm and about 66mm. The width in a lateral direction of the first dual layer area, i.e., the distance between the first lateral edge 623 of the second discrete substrate 620 and the inner lateral edge 614 of the first discrete substrate 610 may be between about 4mm and about 24mm.

As shown in Figs. 3 and 4, looking down on a top view of the body facing side of an embodiment of an absorbent article having the multi-component topsheet 600 and leg cuffs 3401 and 3402, the second discrete substrate 620 is disposed above the first discrete substrate 610 and the third discrete substrate 630. Accordingly, the overlap between the first discrete substrate 610 and the second discrete substrate 620 includes the garment facing side of the second discrete substrate contacting the body facing side of the first discrete substrate. Likewise, the overlap between the third discrete substrate 630 and the second discrete substrate 620 includes the garment facing side of the second discrete substrate contacting the body facing side of the third discrete substrate. Alternatively, as shown in Fig. 5, in an embodiment of an absorbent article having the multi-component topsheet 600 and leg cuffs 3401 and 3402, the second discrete substrate 620 may be disposed below the first discrete substrate 610 and the third discrete substrate 630. Accordingly, the overlap between the first discrete substrate 610 and the second discrete substrate 620 includes the body facing side of the second discrete substrate contacting the garment facing side of the first discrete substrate. Likewise, the overlap between the third discrete substrate 630 and the second discrete substrate 620 includes the body facing side of the second discrete substrate contacting the garment facing side of the third discrete substrate.

The first discrete substrate 610, the second discrete substrate 620, and/or the third discrete substrate 630 may be composed of any one or more of the three-dimensional substrates detailed herein. In some embodiments, the second discrete substrate 620 is composed of an embodiment of the three-dimensional substrates detailed herein, and both the first discrete substrate 610 and the third discrete substrate 630 are made of traditional topsheet materials having no surface modification. In some embodiments, both the first discrete substrate 610 and the third discrete substrate 630 are composed of the same material, but in other embodiments, the first and second discrete substrates may be composed of different materials.

In some embodiments of multi-component topsheet 600, there may be a color difference between the first discrete substrate 610, the second discrete substrate 620, and/or the third discrete substrate 630.

In some embodiments, the single layer of substrate may comprise about 80% or more of the total area of the multi-component topsheet, and the dual layer of substrate may comprise about 20% or less of the total area of the multi-component topsheet. In other embodiments, the single layer of substrate may comprise about 70% or more, about 75% or more, about 85% or more, about 90% or more, or about 95% or more of the total area of the multi-component topsheet, and the dual layer of substrate may comprise about 30% or less, about 25% or less, about 15% or less, about 10% or less, or about 5% or less of the total area of the multi-component topsheet.

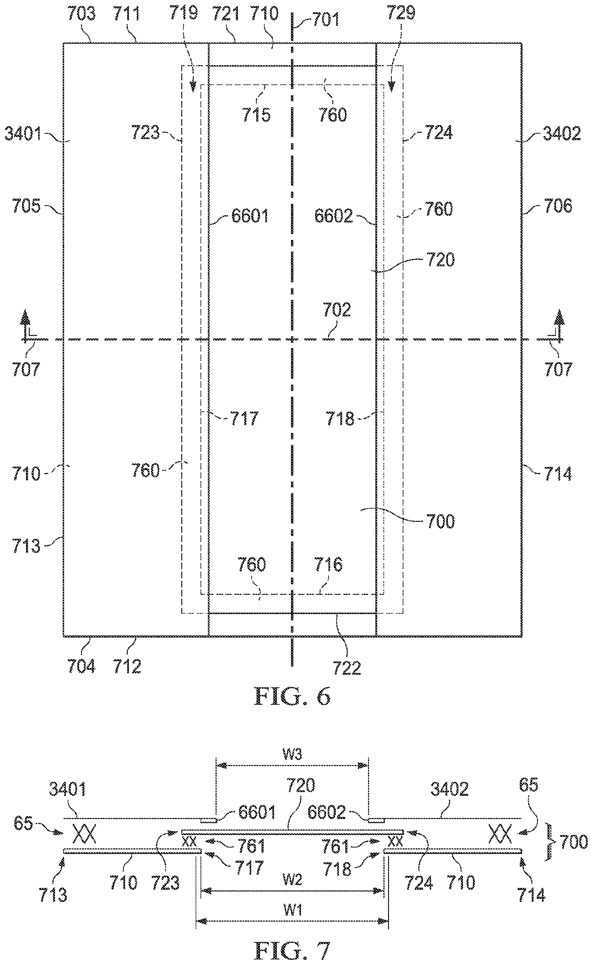

Figs. 6-11 depict top and cross-sectional schematic illustrations of embodiments of absorbent articles according to the present disclosure. For the purpose of convenience of illustrations and descriptions of the present invention, only a multi-component topsheet and part of leg cuffs are illustrated, and other elements and/or layers of absorbent articles are omitted.

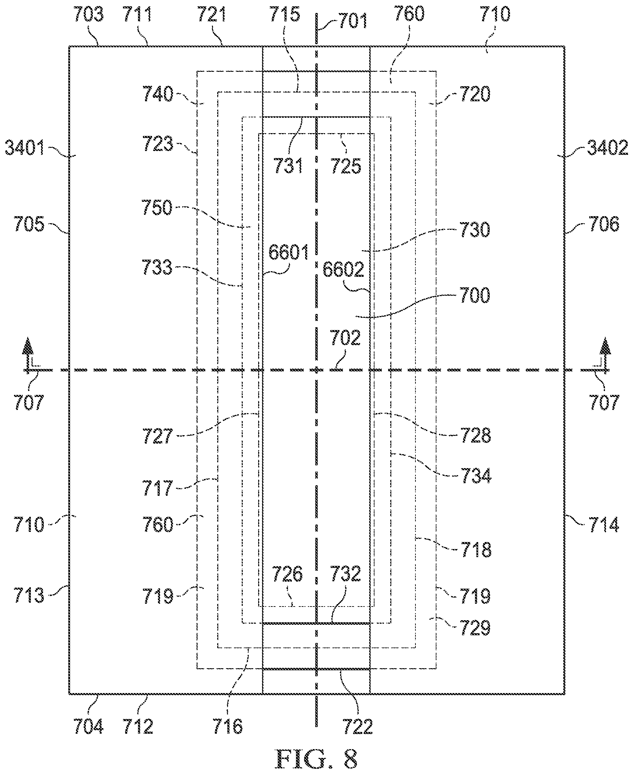

The multi-component topsheet 700 may include a first discrete substrate 710 and a second discrete substrate 720, and optionally in some embodiments (e.g., Figs. 8-11), a third discrete substrate 730. The substrates are joined by any method known in the art (mechanical bonding, hydroentangling, embossing, adhesive bonding, pressure bonding, heat bonding, ultrasonic bonding or by other methods of joining multiple discrete substrates) and in some particular embodiments, by mechanical bonding 761,762. The multi-component topsheet 700 has a longitudinal axis 701 that runs the longer overall dimension of the topsheet (in this case, the MD or machine direction), and a lateral axis 702 that runs perpendicular to the longitudinal axis. The multi-component topsheet 700 may have an overall outer perimeter defined by first longitudinal edge 703, second longitudinal edge 704, first lateral edge 705, and second lateral edge 706. The first discrete substrate may have an outer perimeter defined by first longitudinal edge 711, second longitudinal edge 712, first lateral edge 713, and second lateral edge 714. The first discrete substrate may have an inner perimeter defined by first longitudinal edge 715, second longitudinal edge 716, first lateral edge 717, and second lateral edge 718. The second discrete substrate may have an outer perimeter defined by first longitudinal edge 721, second longitudinal edge 722, first lateral edge 723, and second lateral edge 724. The second discrete substrate may have an inner perimeter defined by first longitudinal edge 725, second longitudinal edge 726, first lateral edge 727, and second lateral edge 728. In embodiments that include a third discrete substrate 730, the third discrete substrate may have a perimeter defined by first longitudinal edge 731, second longitudinal edge 732, first lateral edge 733, and second lateral edge 734.

The outer perimeter of the first discrete substrate 710 may form about 80% or more of the overall outer perimeter of the multi-component topsheet 700, and about 80% or more of the outer perimeter of the second discrete substrate 720 may be joined with a portion of the first discrete substrate. In some embodiments, the outer perimeter of the first discrete substrate 710 may comprise about 70% or more, about 75% or more, about 85% or more, about 90% or more, about 95% or more, or 100% of the overall outer perimeter of the multi-component topsheet 700. In some embodiments, about 70% or more, about 75% or more, about 85% or more, about 90% or more, about 95% or more, or 100% of the outer perimeter of the second discrete substrate 720 may be joined with a portion of the first discrete substrate 710.

In some embodiments, the first and/or second longitudinal edges of the first discrete substrate 710 and/or the second discrete substrate 720 will be common with the longitudinal edges 703, 704 of the multi-component topsheet 700. In some embodiments, the first and/or second lateral edges of the first discrete substrate 710, second discrete substrate 720, and/or the third discrete substrate 730 will be common with the lateral edges 705, 706 of the multi-component topsheet 700. In the non-limiting embodiments of Figs. 6-10, the longitudinal edges 703 and 704 of the multi-component topsheet 700 are common with the longitudinal edges 711 and 712 of the first discrete substrate 710, respectively.

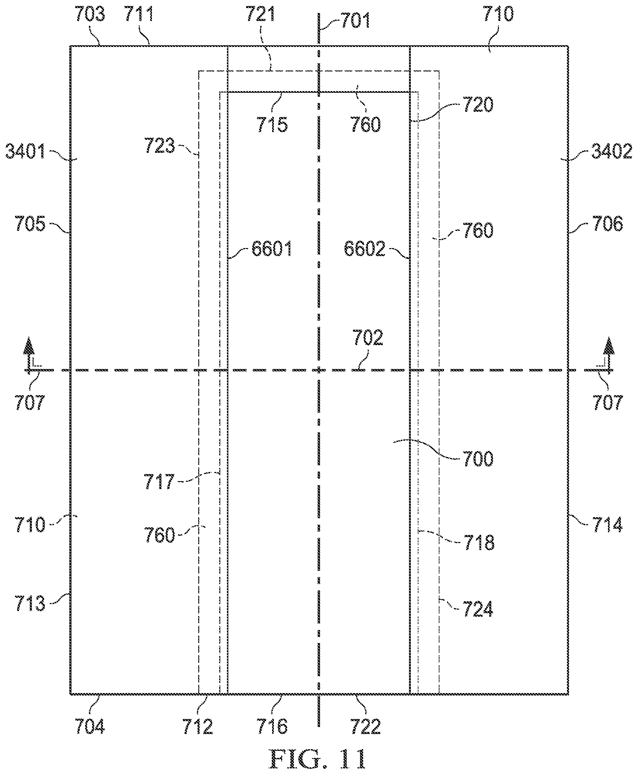

In Figs. 6-10, the lateral edges 705 and 706 of the multi-component topsheet 700 are common with the lateral edges 713 and 714 of the first discrete substrate 710, respectively. In the non-limiting embodiment of Fig. 11, the longitudinal edge 703 of the multi-component topsheet 700 is common with the longitudinal edge 711 of the first discrete substrate 710, and the longitudinal edge 704 of the multi-component topsheet 700 is common with the longitudinal edges 712 and 722 of the first and second discrete substrates. In Fig. 11 the lateral edges 705 and 706 of the multi-component topsheet 700 are common with the lateral edges 713 and 714 of the first discrete substrate 710, respectively.

Still referring to the embodiments depicted in Figs. 6-11 the overlap between the first discrete substrate 710 and the second discrete substrate 720 is the dual layer of substrate for the multi-component topsheet 700, and the areas of the multi-component topsheet 700 that are not the dual layer of substrate are defined as a single layer of substrate. As a non-limiting example, utilizing the embodiment shown in Figs. 6 and 7, if the first single layer area is $120cm^2$ and the second single layer area is $80cm^2$, the single layer of substrate for the multi-component topsheet 700 is $200cm^2$.

Referring now to the embodiments of multi-component topsheet 700 with a third discrete substrate 730 as depicted in Figs. 8-10, multi-component topsheet 700 has overlaps between the first discrete substrate 710 and the second discrete substrate 720, and between the second discrete substrate 720 and the third discrete substrate 730.

Referring to the non-limiting embodiments depicted in Figs. 6-11, the multi-component topsheet 700 may be symmetric along the longitudinal axis 701. The distance between the first lateral edge 705 of the first discrete substrate 710 and the inner perimeter first lateral edge 717 of the first discrete substrate 710 may be between about 20mm and about 70mm. The distance between the first lateral edge 713 of the first discrete substrate 710 and the first lateral edge 723 of the second discrete substrate 720 may be between about 16mm and about 66mm. The distance between the first lateral edge 723 of the second discrete substrate 720 and the second lateral edge 724 of the second discrete substrate 720 may be between about 40mm and about 120mm. The distance between the first lateral edge 723 of the second discrete substrate 720 and the inner perimeter first lateral edge 717 of the first discrete substrate 710 may be between about 4mm and about 24mm.

The first discrete substrate 710, the second discrete substrate 720, and/or the optional third discrete substrate 730 may be composed of a three-dimensional substrate. In some embodiments, the second discrete substrate 720 is composed of any one or more of the three-dimensional substrate.

When viewed from the top, the multi-component topsheets 600, 700 may have one or more shapes, patterns or other distinct visible interfaces between the first discrete substrate 610, 710 and the second discrete substrate 720 (and in certain embodiments, between the second discrete substrate and the third discrete substrate 630, 730).

In some embodiments of multi-component topsheets 600, 700, one or more elastics may be disposed in the overlap between first discrete substrate and second discrete substrate and/or the overlap between second discrete substrate and third discrete substrate.

In some embodiments, the multi-component topsheets 600, 700 may be combined with additional absorbent article elements such as acquisition layers, distributions layers, absorbent layers, etc.

Disposition of Topsheet and Leg Cuffs

An absorbent article according to the present invention comprises a multi-component topsheet and a pair of leg cuff. The topsheet comprises a first discrete substrate having an inner lateral edge in a longitudinal direction, a second discrete substrate, and a third discrete substrate having an inner lateral edge in a longitudinal direction, the second discrete substrate being disposed at least partially intermediate the first discrete substrate and the third discrete substrate, so that the inner lateral edges of the first and second discrete substrates at least partially overlap the second discrete substrate, the second discrete substrate being joined to the first discrete substrate in a first bonding zone and the second discrete substrate being joined to the third discrete substrate in a second bonding zone. The leg cuffs extend at least partially between a front edge and a back edge of the absorbent article on opposite sides of the longitudinal axis of the topsheet, a first and a second leg cuffs being joined to the topsheet and/or the backsheet, the first leg cuff having a first free terminal edge in a longitudinal direction, the second leg cuff having a second free terminal edge in a longitudinal direction. In the absorbent article, the distance between the first free terminal edge and the second free terminal edge is not longer than the distance between an inner edge of the first bonding zone and an inner edge of the second bonding zone in a flat-out state. Or, in the absorbent article of the present invention, the distance between the first free terminal edge and the second free terminal edge is not longer than the distance between the inner lateral edge of the first discrete substrate and the inner lateral edge of the third discrete substrate in a flat-out state.

When the absorbent article of the present invention absorbent article further comprises an additional layer such as an acquisition layer and a distribution layer between the topsheet and the absorbent core, the additional layer may be disposed such a way that lateral edges of the additional layer are located outside of the inner lateral edge of the first discrete substrate and the inner lateral edge of the second discrete substrate.

In some embodiments, referring to Figs. 3-5, absorbent articles according to the present invention comprise a multi-component topsheet 600 comprising a first discrete topsheet 610, a second discrete substrate 620 and a third discrete substrate 630, a first leg cuff 3401 having a first free terminal edge 6601, and a second leg cuff 3402 having a second free terminal edge 6602. In one embodiment, the distance W3 between the first free terminal edge 6601 and the second free terminal edge 6602 is not longer than the distance W1 between an inner edge of the first bonding zone 619 and an inner edge of the second bonding zone 629 in a flat-out state. W3 may be about 8mm to 12mm shorter than W1. The first and second bonding zones, 619 and 629, have inner edges in a longitudinal direction. The inner edge of each of the first and second bonding zones 619 and 612 means that an edge in a longitudinal direction of the boding zone closer to the longitudinal axis of the topsheet. When the bonding zone 619, 629 has a plurality of bondings, an inner edge of the bonding zone means an inner edge of a bonding which is closest to the longitudinal axis of the topsheet 600. Widths of bonding zone 619, 629 may be the same as or shorter to width of a first dual layer area 640 and a second dual layer area 650 explained below, respectively. In another embodiment, still referring to Figs. 3-5, the distance W3 between the first free terminal edge 6601 of the first leg cuff 3401 and the second free terminal edge 6602 of the second leg cuff 3402 may not be longer than the distance W2 between the inner lateral edge 614 of the first discrete substrate 610 and the inner lateral edge 633 of the third discrete substrate 630 in a flat-out state.

In some other embodiments, referring to Figs. 6, 7 and 11, absorbent articles according to the present invention comprise a multi-component topsheet 700 comprising a first discrete topsheet 710 and a second discrete substrate 720, a first leg cuff 3401 having a first free terminal edge 6601, and a second leg cuff 3402 having a second free terminal edge 6602. In one embodiment, the distance W3 between the first free terminal edge 6601 and the second free terminal edge 6602 is not longer than the distance W1 between an inner edge of the first bonding zone 719 and an inner edge of the second bonding zone 729 in a flat-out state. In another embodiment, still referring to Figs. 6 and 7, the distance W3 between the first free terminal edge 6601 of the first leg cuff 3401 and the second free terminal edge 6602 of the second leg cuff 3402 may not be longer than the distance W2 between the inner perimeter first lateral edge 717 and the inner perimeter second lateral edge 718 of the first discrete substrate 710 in a flat-out state. Descriptions for bonding zones disclosed with respect to Figs. 3-5 are also applied for Figs. 6, 7 and 11.

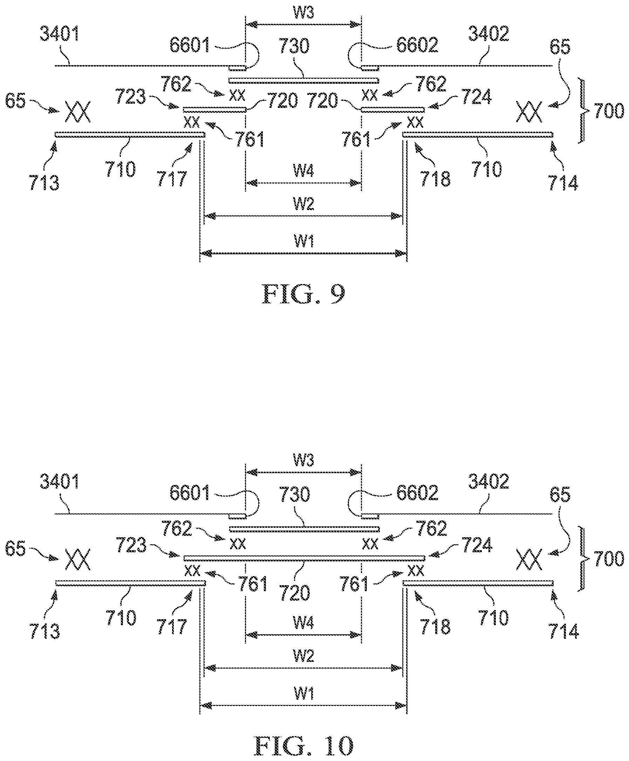

In some other embodiments, referring to Figs. 8-10, absorbent articles according to the present invention comprise a multi-component topsheet 700 comprising a first discrete topsheet 710, a second discrete substrate 720 and a third discrete substrate 730, a first leg cuff 3401 having a first free terminal edge 6601, and a second leg cuff 3402 having a second free terminal edge 6602. The first discrete substrate 710 is joined to the second discrete substrate 720 in a first bonding zone 719 and to a second bonding zone 729 opposite to the first bonding zone 719 about the longitudinal axis, and the second discrete substrate 720 is joined to the third discrete substrate 730 in a third bonding zone 739 and to a fourth bonding zone 749 opposite to the third bonding zone 739 about the longitudinal axis. In one embodiment, the distance W3 between the first free terminal edge 6601 and the second free terminal edge 6602 is not longer than the distance W1 between an inner edge of the firs bonding zone 719 and an inner edge of the second bonding zone 729 in a flat-out state. Or, the distance W3 between the first free terminal edge 6601 and the second free terminal edge 6602 is not longer than the distance W2 between the inner perimeter first lateral edge 717 and the inner perimeter second lateral edge 718 of the first discrete substrate 710 in a flat-out state. In another embodiment, still referring to Figs. 8 and 9, the distance W4 between the first free terminal edge 6601 of the first leg cuff 3401 and the second free terminal edge 6602 of the second leg cuff 3402 may not be longer than the distance W2 between the inner perimeter first lateral edge 727 and the inner perimeter second lateral edge 728 of the first discrete substrate 720 in a flat-out state. Descriptions for bonding zones disclosed with respect to Figs. 3-5 are also applied for Figs. 8-10.

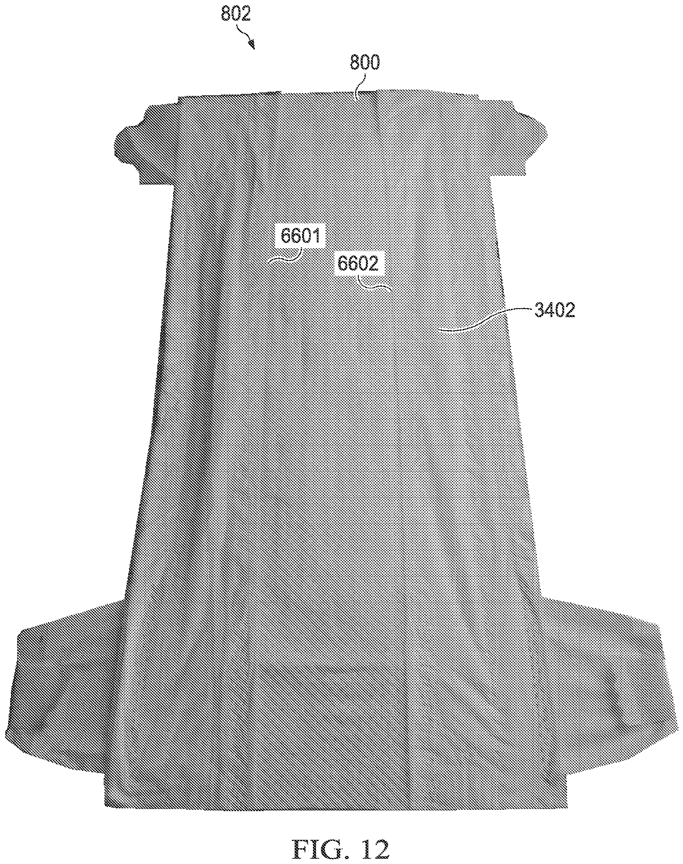

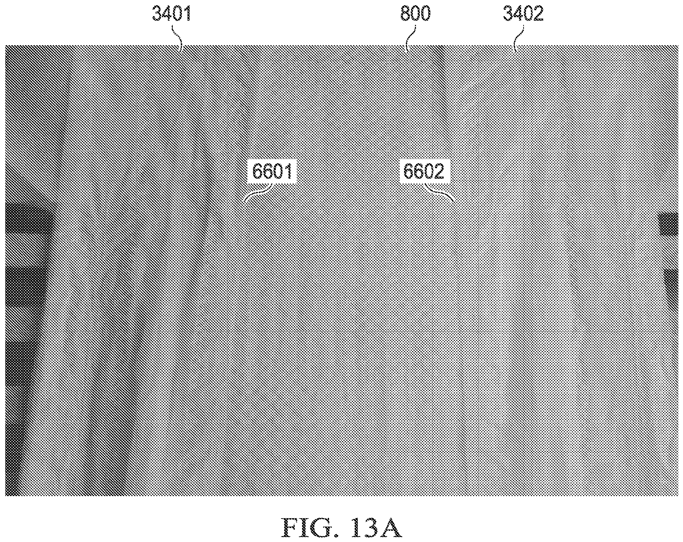

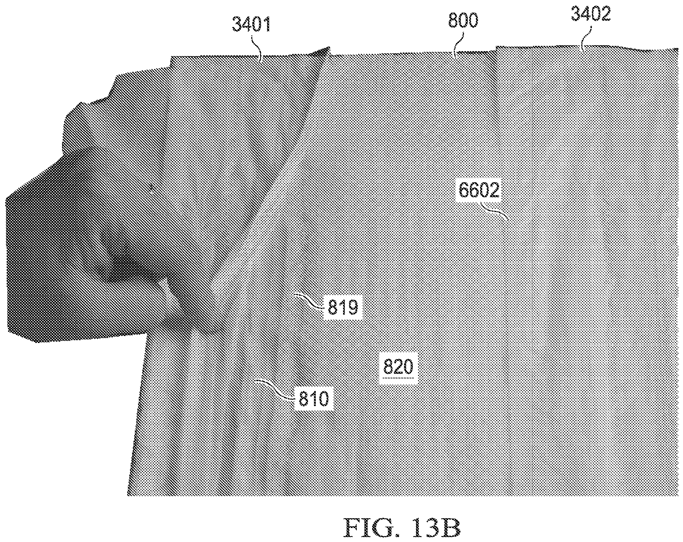

Referring to Figs. 12, 13A and 13B, a multi-component topsheet 800 and leg cuffs 3401, 3402 are illustrated an on absorbent article 802. Fig. 12 is a perspective view of the absorbent article 802 with the wearer-facing surface facing the viewer. Figs. 13A and 13B are enlarged top views of a portion of the absorbent article 802. As shown in Figs. 12 and 13A, leg cuffs 3401 and 3402 extend towards a longitudinal axis of the absorbent article so that bonding areas in the multi-component topsheet is not perceived by users in a flat-out state. Fig. 13B shows a top view of a portion of the absorbent article 802 when a bonding zone 819 where the second discrete substrate 820 of the multi-component topsheet 800 joins the third discrete substrate 810 and the first lateral edge 823 of the second discrete substrate 820 are exposed.

In another embodiment, where the absorbent article has an acquisition layer below the topsheet, the distance between two free terminal edges of a pair of leg cuffs is shorter than the distance between two free terminal edges of leg cuffs in a plan view of the absorbent article when the absorbent article is unfolded.

Any portion of the multi-component topsheet suitable for the present invention may be coated with a lotion, a skin care composition, and/or antibacterial agents in a lotion area as is generally disclosed in the art. The distance between the inner bonding edge of the first bonding area and a lateral edge of the lotion area adjacent to the inner bonding edge of the first bonding area may be at least 4mm. If lotion is located too close to a free terminal edge of a leg cuff, it may dissolve adhesive used to adhere elastic elements in the leg cuff near the free terminal edge.

For the purpose of providing holistic material integrity when customers unfold the absorbent article, the basis weight and/or opacity of the materials for the topsheet and leg cuffs can be carefully determined. In one embodiment, when the basis weight of the first discrete substrate is BF, the basis weight of the second discrete substrate is BS and the basis weight of the leg cuff is BL, the difference between BS and BS+BL is no more than about 15gsm. For example, the basis weight of the second discrete substrate is about 20-50gsm, and the total basis weight of the first discrete substrate and the leg cuff is about 27-42gsm.

In another embodiment, when the opacity of the first discrete substrate is OF, the opacity of the second discrete substrate is OS and the opacity of the leg cuff is OL, the difference between OS and OF+OL is no more than about 10 (20)%.

Measurement of Opacity

Opacity may be measured using a 0° illumination / 45° detection, circumferential optical geometry, spectrophotometer with a computer interface such as the HunterLab LabScan XE running Universal Software (available from Hunter Associates Laboratory Inc., Reston, VA, US). Instrument calibration and measurements are made using the standard white and black calibration plates provided by the vendor. All testing is performed in a room maintained at about 23 $\pm$ 2 °C and about 50 $\pm$ 5 % relative humidity.

The spectrophotometer is configured for the XYZ color scale, D65 illuminant, 10° standard observer, with UV filter set to nominal. The instrument is standardized according to the manufacturer's procedures using the 44.45 mm (1.750 inch) area view. After calibration, the software is set to the Y opacity procedure which prompts the operator to cover the sample with either the white or black calibration tile during the measurement.

To obtain a specimen, lay the sample flat on a bench, body facing surface downward, and 101.6 mm by 101.6 mm portions of sample are cut using scissor for analysis. When the sample is a combined material, they are overlayed to obtain a specimen. Samples are pre-conditioned at 23 °C $\pm$ 2 C° and 50% $\pm$ 5% relative humidity for two hours prior to testing.

Place specimen over the measurement port. The specimen should completely cover the port with the surface corresponding to the garment-facing surface of the article directed toward the port. Cover the specimen with the white standard plate. Take a reading, then remove the white tile and replace it with the black standard tile without moving the specimen. Obtain a second reading, and calculate the opacity as follows:

$$\text{Opacity} = (\text{Y value}_{\text{(black backing)}} / \text{Y value}_{\text{(white backing)}}) \times 100$$

A total of three identical material, or materials combined, are analyzed and their opacity results recorded. Calculate and report the average opacity to the nearest 0.1%.

**Claims**

1. An absorbent article (20) comprising a front edge (10) and a back edge (12), the absorbent article (20) comprising:

   a) a multi-component topsheet (24) having a longitudinal axis (80), the topsheet (24) comprising:

      i) a first discrete substrate (610) having an inner lateral edge (614);
      ii) a second discrete substrate (620); and
      iii) a third discrete substrate (630) having an inner lateral edge (633);

   wherein the second discrete substrate (620) is disposed at least partially intermediate the first discrete substrate (610) and the third discrete substrate (630) in a longitudinal direction, so that the inner lateral edges (614, 633) of the first and second discrete substrates (610, 630) at least partially overlap the second discrete substrate (620),

      wherein the second discrete substrate (620) is joined to the first discrete substrate (610) in a first bonding zone (619), the first bonding zone (619) having an inner edge;
      wherein the second discrete substrate (620) is joined to the third discrete substrate (630) in a second bonding zone (629), the second bonding zone (629) having an inner edge; and

   b) a backsheet (25);

c) an absorbent core (28) at least partially intermediate the backsheet (25) and the topsheet (24); and

d) a pair of leg cuffs (34) extending at least partially between the front edge (10) and the back edge of the absorbent article (20) on opposite sides of the longitudinal axis (80) of the topsheet (24), each leg cuff joined to the topsheet (24) or the backsheet (25), a first leg cuff (3401) and a second leg cuff (3402) having a first free terminal edge (6601) and a second free terminal edge (6602) in a longitudinal direction, respectively,

wherein the distance between the first free terminal edge (6601) and the second free terminal edge (6602) is not longer than the distance between the inner edge of the first bonding zone (619) and the inner edge of the second bonding zone (629) in a flat-out state.

2. The absorbent article (20) of claim 1, wherein the distance between the first free terminal edge (6601) of the first leg cuff (3401) and the second free terminal edge (6602) of the second leg cuff (3402) is not longer than the distance between the inner lateral edge (614) of the first discrete substrate (610) and the inner lateral edge (633) of the third discrete substrate (630).

3. The absorbent article (20) of claim 1, wherein the distance between the first free terminal edge (6601) and the second free terminal edge (6602) is about 8mm to 12mm shorter than the distance between an inner edge of the first bonding zone (619) and an inner edge of the second bonding zone (629).

4. The absorbent article (20) according to any of the preceding claims, wherein the second discrete substrate (620) is joined to the first discrete substrate (610) by a method selected from the group consisting of mechanical bonding, hydroentangling, embossing, adhesive bonding, pressure bonding, heat bonding, ultrasonic bonding and combinations thereof.

5. The absorbent article (20) according to any of the preceding claims, wherein a garment-facing surface of the second discrete substrate (620) faces wearer-facing surfaces of the first discrete substrate (610) and the third discrete substrates (630).

6. The absorbent article (20) according to any of the preceding claims, wherein the first discrete substrate (610) and the third discrete substrate (630) are the same substrate.

7. The absorbent article (20) according to any of the preceding claims, wherein when the basis weight of the first discrete substrate (610) is BF, the basis weight of the second discrete substrate (620) is BS and the basis weight of the leg cuff is BL, the difference between BS and BS+BL is no more than 15 gsm.

8. The absorbent article (20) according to any of the preceding claims, wherein the basis weight of the second discrete substrate (620) is 20-50 gsm, and the total basis weight of the first discrete substrate (610) and the leg cuff is 27-42 gsm.

9. The absorbent article (20) according to any of the preceding claims, wherein when the opacity of the first discrete substrate (610) is OF, the opacity of the second discrete substrate (620) is OS and the opacity of the leg cuff is OL, the difference between OS and OF+OL is no more than 10 %.

10. The absorbent article (20) according to any of the preceding claims, wherein the absorbent article (20) further comprises a lotion applied in a lotion area on the topsheet (24).

11. The absorbent article (20) according to claim 10, wherein the lotion area has lateral edges, and the distance between the inner bonding edge of the first bonding area and a lateral edge of the lotion area adjacent to the inner bonding edge of the first bonding area is at least 4 mm.

12. The absorbent article (20) according to any of the preceding claims, wherein the inner lateral edge (614) of the first discrete substrate (610) is substantially symmetrical to the inner lateral edge (633) of the third discrete substrate (630) about the longitudinal axis (80) of the topsheet (24).

13. The absorbent article (20) according to any of the preceding claims, wherein the second discrete substrate (620) is more hydrophobic than the first discrete substrate (610).

14. The absorbent article (20) of any of the preceding claims,, the absorbent article (20) further comprising an additional layer disposed between the topsheet (24) and the absorbent core (28), wherein the additional layer is disposed in such a way that lateral edges of the additional layer are located outside of the inner lateral edge (614) of the first discrete substrate (610) and the inner lateral edge of the second discrete substrate (620).

15. The absorbent article (20) according to any of the preceding claims, wherein the second discrete substrate (620) comprises an element selected from the group consisting of a plurality of projections, a plurality of recesses and a plurality of apertures and combinations thereof.

16. An absorbent article (20) comprising a front edge (10) and a back edge (12), the absorbent article (20) comprising:

a) a multi-component topsheet (700) having a longitudinal axis (701), the topsheet (24) comprising:

i) a first discrete substrate (710) forming 80% or more of an outer perimeter of the topsheet (24); and
ii) a second discrete substrate (720) wherein 80% or more of an outer perimeter of the second discrete substrate (720) is joined to the first discrete substrate (710);

wherein the first discrete substrate (710) is joined to the second discrete substrate (720) in a first bonding zone (719) and to a second bonding zone (729) opposite to the first bonding zone (719) about the longitudinal axis (701);

wherein the topsheet (700) has a single layer of substrate in about 75% or more of the total area of the topsheet (700) and a dual layer of substrate in about 25% or less of the total area of the topsheet (700), wherein the dual layer of substrate is formed from an overlap between the first discrete substrate (710) and the second discrete substrate (720); and

b) a backsheet (25);
c) an absorbent core (28) at least partially intermediate the backsheet (25) and the topsheet (700); and
d) a pair of leg cuffs (34) extending at least partially between the front edge (10) and the back edge of the absorbent article (20) on opposite sides of the longitudinal axis (701) of the topsheet (700), each leg cuff joined to the topsheet (700) or the backsheet (25), a first leg cuff (3401) and a second leg cuff (3402) having a first free terminal edge (6601) and a second free terminal edge (6602) in a longitudinal direction, respectively,

wherein the distance between the first free terminal edge (6601) and the second free terminal edge (6602) is not longer than the distance between an inner edge of the first bonding zone (719) and an inner edge of the second bonding zone (729) in a flat-out state.

17. The absorbent article (20) of claim 16, wherein the first discrete substrate (710) have a first and second inner lateral edges (713, 714) overlapping the second discrete substrate (720), wherein the distance between the first free terminal edge (6601) of the first leg cuff (3401) and the second free terminal edge (6602) of the second leg cuff (3402) is shorter than the distance between the first and second inner lateral edges (713, 714) of the first discrete substrate (710).

18. The absorbent article (20) of claim 16, wherein the second discrete substrate (720) comprises an element selected from the group consisting of a plurality of projections, a plurality of recesses and a plurality of apertures and combinations thereof.

19. The absorbent article (20) according to any of claims 16-18, wherein the dual area has a distance of 8 to 20 mm.

**Patentansprüche**

1. Absorptionsartikel (20), umfassend einen vorderseitigen Rand (10) und einen rückseitigen Rand (12), wobei der Absorptionsartikel (20) umfasst:

a) eine Mehrkomponenten-Oberschicht (24), die eine Längsachse (80) aufweist, wobei die Oberschicht (24) umfasst:

i) ein erstes gesondertes Substrat (610), das einen inneren Seitenrand (614) aufweist;

ii) ein zweites gesondertes Substrat (620); und

iii) ein drittes gesondertes Substrat (630), das einen inneren Seitenrand (633) aufweist;

wobei das zweite gesonderte Substrat (620) mindestens teilweise zwischen dem ersten gesonderten Substrat (610) und dem dritten gesonderten Substrat (630) in einer Längsrichtung angeordnet ist, so dass die inneren Seitenränder (614, 633) des ersten und zweiten gesonderten Substrats (610, 630) mindestens teilweise das zweite gesonderte Substrat (620) überlappen,

wobei das zweite gesonderte Substrat (620) mit dem ersten gesonderten Substrat (610) in einer ersten Verbindungszone (619) verbunden ist, wobei die erste Verbindungszone (619) einen inneren Seitenrand aufweist;

wobei das zweite gesonderte Substrat (620) mit dem dritten gesonderten Substrat (630) in einer zweiten Verbindungszone (629) verbunden ist, wobei die zweite Verbindungszone (629) einen inneren Seitenrand aufweist; und

b) eine Unterschicht (25);

c) einen Absorptionskern (28) mindestens teilweise zwischen der Unterschicht (25) und der Oberschicht (24); und

d) ein Paar Beinbündchen (34), die sich mindestens teilweise zwischen dem vorderseitigen Rand (10) und dem rückseitigen Rand des Absorptionsartikels (20) auf gegenüberliegenden Seiten der Längsachse (80) der Oberschicht (24) erstrecken, wobei jedes Beinbündchen mit der Oberschicht (24) oder der Unterschicht (25) verbunden ist, wobei ein erstes Beinbündchen (3401) und ein zweites Beinbündchen (3402) einen ersten freien endständigen Rand (6601) und einen zweiten freien endständigen Rand (6602) jeweils in einer Längsrichtung aufweisen,

wobei der Abstand zwischen dem ersten freien endständigen Rand (6601) und dem zweiten freien endständigen Rand (6602) nicht länger als der Abstand zwischen dem inneren Rand der ersten Verbindungszone (619) und dem inneren Rand der zweiten Verbindungszone (629) in einem flach ausgelegten Zustand ist.

2. Absorptionsartikel (20) nach Anspruch 1, wobei der Abstand zwischen dem ersten freien endständigen Rand (6601) des ersten Beinbündchens (3401) und dem zweiten freien endständigen Rand (6602) des zweiten Beinbündchens (3402) nicht länger als der Abstand zwischen dem inneren Seitenrand (614) des ersten gesonderten Substrats (610) und dem inneren Seitenrand (633) des dritten gesonderten Substrats (630) ist.

3. Absorptionsartikel (20) nach Anspruch 1, wobei der Abstand zwischen dem ersten freien endständigen Rand (6601) und dem zweiten freien endständigen Rand (6602) etwa 8 mm bis 12 mm kürzer als der Abstand zwischen einem inneren Rand der ersten Verbindungszone (619) und einem inneren Rand der zweiten Verbindungszone (629) ist.

4. Absorptionsartikel (20) nach einem der vorstehenden Ansprüche, wobei das zweite gesonderte Substrat (620) mit dem ersten gesonderten Substrat (610) durch ein Verfahren, ausgewählt aus der Gruppe bestehend aus mechanischer Verbindung, Wasserstrahlverfestigung, Prägen, Adhäsionsverbindung, Druckverbindung, Heißkleben, Ultraschallverbindung und Kombinationen davon, verbunden ist.

5. Absorptionsartikel (20) nach einem der vorstehenden Ansprüche, wobei eine bekleidungsseitige Oberfläche des zweiten gesonderten Substrats (620) trägerseitigen Oberflächen des ersten gesonderten Substrats (610) und des dritten gesonderten Substrats (630) zugewandt ist.

6. Absorptionsartikel (20) nach einem der vorstehenden Ansprüche, wobei das erste gesonderte Substrat (610) und das dritte gesonderte Substrat (630) dasselbe Substrat sind.

7. Absorptionsartikel (20) nach einem der vorstehenden Ansprüche, wobei, wenn das Basisgewicht des ersten gesonderten Substrats (610) BF ist, das Basisgewicht des zweiten gesonderten Substrats (620) BS ist und das Basisgewicht des Beinbündchens BL ist, der Unterschied zwischen BS und BS+BL nicht mehr als 15 Gramm pro Quadratmeter beträgt.

8. Absorptionsartikel (20) nach einem der vorstehenden Ansprüche, wobei das Basisgewicht des zweiten gesonderten Substrats (620) 20-50 Gramm pro Quadratmeter beträgt, und das Gesamtbasisgewicht des ersten gesonderten Substrats (610) und der Beinbündchen 27-42 Gramm pro Quadratmeter beträgt.

9. Absorptionsartikel (20) nach einem der vorstehenden Ansprüche, wobei, wenn die Trübung des ersten gesonderten Substrats (610) OF ist, die Trübung des zweiten gesonderten Substrats (620) OS ist und die Trübung des Beinbündchens OL ist, der Unterschied zwischen OS und OF+OL nicht mehr als 10 % beträgt.

10. Absorptionsartikel (20) nach einem der vorstehenden Ansprüche, wobei der Absorptionsartikel (20) ferner eine Lotion umfasst, die in einer Lotionsfläche auf die Oberschicht (24) aufgebracht ist.

11. Absorptionsartikel (20) nach Anspruch 10, wobei die Lotionsfläche Seitenränder aufweist und der Abstand zwischen dem inneren Verbindungsrand der ersten Verbindungsfläche und einem Seitenrand der Lotionsfläche benachbart zum inneren Verbindungsrand der ersten Verbindungsfläche mindestens 4 mm beträgt.

12. Absorptionsartikel (20) nach einem der vorstehenden Ansprüche, wobei der innere Seitenrand (614) des ersten gesonderten Substrats (610) im Wesentlichen symmetrisch zu dem inneren Seitenrand (633) des dritten gesonderten Substrats (630) um die Längsachse (80) der Oberschicht (24) ist.

13. Absorptionsartikel (20) nach einem der vorstehenden Ansprüche, wobei das zweite gesonderte Substrat (620) hydrophober ist als das erste gesonderte Substrat (610).

14. Absorptionsartikel (20) nach einem der vorstehenden Ansprüche, wobei der Absorptionsartikel (20) ferner eine zusätzliche Schicht umfasst, die zwischen der Oberschicht (24) und dem Absorptionskern (28) angeordnet ist, wobei die zusätzliche Schicht auf derartige Weise angeordnet ist, dass Seitenränder der zusätzlichen Schicht außerhalb des inneren Seitenrands (614) des ersten gesonderten Substrats (610) und des inneren Seitenrands des zweiten gesonderten Substrats (620) angeordnet sind.

15. Absorptionsartikel (20) nach einem der vorstehenden Ansprüche, wobei das zweite gesonderte Substrat (620) ein Element ausgewählt aus der Gruppe bestehend aus einer Vielzahl von Vorsprüngen, einer Vielzahl von Aussparungen und einer Vielzahl von Öffnungen und Kombinationen davon, umfasst.

16. Absorptionsartikel (20), umfassend einen vorderseitigen Rand (10) und einen rückseitigen Rand (12), wobei der Absorptionsartikel (20) umfasst:

   a) eine Mehrkomponenten-Oberschicht (700), die eine Längsachse (701) aufweist, wobei die Oberschicht (24) umfasst:

      i) ein erstes gesondertes Substrat (710), das 80 % oder mehr eines Außenumfangs der Oberschicht (24) bildet; und
      ii) ein zweites gesondertes Substrat (720), wobei 80 % oder mehr eines Außenumfangs des zweiten gesonderten Substrats (720) mit dem ersten gesonderten Substrat verbunden sind (710);

   wobei das erste gesonderte Substrat (710) mit dem zweiten gesonderten Substrat (720) in einer ersten Verbindungszone (719) und mit einer zweiten Verbindungszone (729) gegenüber der ersten Verbindungszone (719) um die Längsachse (701) herum verbunden ist;

      wobei die Oberschicht (700) eine einzelne Substratschicht in etwa 75 % oder mehr der Gesamtfläche der Oberschicht (700) und eine doppelte Substratschicht in etwa 25 % oder weniger der Gesamtfläche der Oberschicht (700) aufweist,
      wobei die doppelte Substratschicht aus einer Überlappung zwischen dem ersten gesonderten Substrat (710) und dem zweiten gesonderten Substrat (720) gebildet ist; und

   b) eine Unterschicht (25);
   c) einen Absorptionskern (28) mindestens teilweise zwischen der Unterschicht (25) und der Oberschicht (700); und
   d) ein Paar Beinbündchen (34), die sich mindestens teilweise zwischen dem vorderseitigen Rand (10) und dem rückseitigen Rand des Absorptionsartikels (20) auf gegenüberliegenden Seiten der Längsachse (701) der Oberschicht (700) erstrecken, wobei jedes Beinbündchen mit der Oberschicht (700) oder der Unterschicht (25) verbunden ist, wobei ein erstes Beinbündchen (3401) und ein zweites Beinbündchen (3402) einen ersten freien endständigen Rand (6601) und einen zweiten freien endständigen Rand (6602) jeweils in einer Längsrichtung aufweisen,

wobei der Abstand zwischen dem ersten freien endständigen Rand (6601) und dem zweiten freien endständigen Rand (6602) nicht länger als der Abstand zwischen einem inneren Rand der ersten Verbindungszone (719) und einem inneren Rand der zweiten Verbindungszone (729) in einem flach ausgelegten Zustand ist.

**17.** Absorptionsartikel (20) nach Anspruch 16, wobei das erste gesonderte Substrat (710) einen ersten und einen zweiten inneren Seitenrand (713, 714) aufweisen, die das zweite gesonderte Substrat (720) überlappen, wobei der Abstand zwischen dem ersten freien endständigen Rand (6601) des ersten Beinbündchens (3401) und dem zweiten freien endständigen Rand (6602) des zweiten Beinbündchens (3402) kürzer ist als der Abstand zwischen dem ersten und dem zweiten inneren Seitenrand (713, 714) des ersten gesonderten Substrats (710).

**18.** Absorptionsartikel (20) nach Anspruch 16, wobei das zweite gesonderte Substrat (720) ein Element ausgewählt aus der Gruppe bestehend aus einer Vielzahl von Vorsprüngen, einer Vielzahl von Aussparungen und einer Vielzahl von Öffnungen und Kombinationen davon, umfasst.

**19.** Absorptionsartikel (20) nach einem der Ansprüche 16 bis 18, wobei die doppelte Fläche einen Abstand von 8 bis 20 mm aufweist.

**Revendications**

**1.** Article absorbant (20) comprenant un bord avant (10) et un bord arrière (12), l'article absorbant (20) comprenant :

a) une feuille de dessus à plusieurs composants (24) ayant un axe longitudinal (80), la feuille de dessus (24) comprenant :

i) un premier substrat distinct (610) ayant un bord latéral interne (614) ;
ii) un deuxième substrat distinct (620) ; et
iii) un troisième substrat distinct (630) ayant un bord latéral interne (633) ;

dans lequel le deuxième substrat distinct (620) est disposé au moins partiellement entre le premier substrat distinct (610) et le troisième substrat distinct (630) dans une direction longitudinale, de sorte que les bords latéraux internes (614, 633) des premier et deuxième substrats distincts (610, 630) chevauchent au moins partiellement le deuxième substrat distinct (620),

dans lequel le deuxième substrat distinct (620) est joint au premier substrat distinct (610) dans une première zone de liaison (619), la première zone de liaison (619) ayant un bord interne ;
dans lequel le deuxième substrat distinct (620) est joint au troisième substrat distinct (630) dans une deuxième zone de liaison (629), la deuxième zone de liaison (629) ayant un bord interne ; et

b) une feuille de fond (25) ;
c) une âme absorbante (28) au moins partiellement entre la feuille de fond (25) et la feuille de dessus (24) ; et
d) une paire de revers de jambe (34) s'étendant au moins partiellement entre le bord avant (10) et le bord arrière de l'article absorbant (20) sur des côtés opposés de l'axe longitudinal (80) de la feuille de dessus (24), chaque revers de jambe joint à la feuille de dessus (24) ou à la feuille de fond (25), un premier revers de jambe (3401) et un deuxième revers de jambe (3402) ayant un premier bord terminal libre (6601) et un deuxième bord terminal libre (6602) dans une direction longitudinale, respectivement,

dans lequel la distance entre le premier bord terminal libre (6601) et le deuxième bord terminal libre (6602) n'est pas plus longue que la distance entre le bord interne de la première zone de liaison (619) et le bord interne de la deuxième zone de liaison (629) dans un état à plat.

**2.** Article absorbant (20) selon la revendication 1, dans lequel la distance entre le premier bord terminal libre (6601) du premier revers de jambe (3401) et le deuxième bord terminal libre (6602) du deuxième revers de jambe (3402) n'est pas plus longue que la distance entre le bord latéral interne (614) du premier substrat distinct (610) et le bord latéral interne (633) du troisième substrat distinct (630).

**3.** Article absorbant (20) selon la revendication 1, dans lequel la distance entre le premier bord terminal libre (6601) et le deuxième bord terminal libre (6602) est d'environ 8 mm à 12 mm plus courte que la distance entre un bord interne de la première zone de liaison (619) et un bord interne de la deuxième zone de liaison (629).

4. Article absorbant (20) selon l'une quelconque des revendications précédentes, dans lequel le deuxième substrat distinct (620) est joint au premier substrat distinct (610) par un procédé choisi dans le groupe constitué de liaison mécanique, enchevêtrement par voie hydraulique, gaufrage, liaison par adhésif, liaison par pression, liaison thermique, liaison par ultrasons et combinaisons de ceux-ci.

5. Article absorbant (20) selon l'une quelconque des revendications précédentes, dans lequel une surface faisant face au vêtement du deuxième substrat distinct (620) fait face à des surfaces faisant face au porteur du premier substrat distinct (610) et du troisième substrat distinct (630).

6. Article absorbant (20) selon l'une quelconque des revendications précédentes, dans lequel le premier substrat distinct (610) et le troisième substrat distinct (630) sont le même substrat.

7. Article absorbant (20) selon l'une quelconque des revendications précédentes, dans lequel lorsque la masse surfacique du premier substrat distinct (610) est BF, la masse surfacique du deuxième substrat distinct (620) est BS et que la masse surfacique du revers de jambe est BL, la différence entre BS et BS+BL n'est pas supérieure à 15 g/m².

8. Article absorbant (20) selon l'une quelconque des revendications précédentes, dans lequel la masse surfacique du deuxième substrat distinct (620) est 20 à 50 g/m², et la masse surfacique totale du premier substrat distinct (610) et du revers de jambe est de 27 à 42 g/m².

9. Article absorbant (20) selon l'une quelconque des revendications précédentes, dans lequel lorsque l'opacité du premier substrat distinct (610) est OF, que l'opacité du deuxième substrat distinct (620) est OS et que l'opacité du revers de jambe est OL, la différence entre OS et OF+OL n'est pas supérieure à 10 %.

10. Article absorbant (20) selon l'une quelconque des revendications précédentes, dans lequel l'article absorbant (20) comprend en outre une lotion appliquée dans une zone de lotion sur la feuille de dessus (24).

11. Article absorbant (20) selon la revendication 10, dans lequel la zone de lotion a des bords latéraux, et la distance entre le bord de liaison interne de la première zone de liaison et un bord latéral de la zone de lotion adjacent au bord de liaison interne de la première zone de liaison est d'au moins 4 mm.

12. Article absorbant (20) selon l'une quelconque des revendications précédentes, dans lequel le bord latéral interne (614) du premier substrat distinct (610) est sensiblement symétrique au bord latéral interne (633) du troisième substrat distinct (630) autour de l'axe longitudinal (80) de la feuille de dessus (24).

13. Article absorbant (20) selon l'une quelconque des revendications précédentes, dans lequel le deuxième substrat distinct (620) est plus hydrophobe que le premier substrat distinct (610).

14. Article absorbant (20) selon l'une quelconque des revendications précédentes, l'article absorbant (20) comprenant en outre une couche supplémentaire disposée entre la feuille de dessus (24) et l'âme absorbante (28), dans lequel la couche supplémentaire est disposée de telle sorte que des bords latéraux de la couche supplémentaire sont situés à l'extérieur du bord latéral interne (614) du premier substrat distinct (610) et du bord latéral interne du deuxième substrat distinct (620).

15. Article absorbant (20) selon l'une quelconque des revendications précédentes, dans lequel le deuxième substrat distinct (620) comprend un élément choisi dans le groupe constitué d'une pluralité de saillies, d'une pluralité d'évidements et d'une pluralité d'ouvertures et de combinaisons de ceux-ci.

16. Article absorbant (20) comprenant un bord avant (10) et un bord arrière (12), l'article absorbant (20) comprenant :

   a) une feuille de dessus à plusieurs composants (700) ayant un axe longitudinal (701), la feuille de dessus (24) comprenant :

   i) un premier substrat distinct (710) formant 80 % ou plus d'un périmètre externe de la feuille de dessus (24) ; et
   ii) un deuxième substrat distinct (720) où 80 % ou plus d'un périmètre externe du deuxième substrat distinct (720) est joint au premier substrat distinct (710) ;

dans lequel le premier substrat distinct (710) est joint au deuxième substrat distinct (720) dans une première zone de liaison (719) et à une deuxième zone de liaison (729) opposée à la première zone de liaison (719) autour de l'axe longitudinal (701) ;

dans lequel la feuille de dessus (700) a une couche unique de substrat d'environ 75 % ou plus de l'aire totale de la feuille de dessus (700) et une double couche de substrat d'environ 25 % ou moins de l'aire totale de la feuille de dessus (700),
dans lequel la double couche de substrat est formée à partir d'un chevauchement entre le premier substrat distinct (710) et le deuxième substrat distinct (720) ; et

b) une feuille de fond (25) ;
c) une âme absorbante (28) au moins partiellement entre la feuille de fond (25) et la feuille de dessus (700) ; et
d) une paire de revers de jambe (34) s'étendant au moins partiellement entre le bord avant (10) et le bord arrière de l'article absorbant (20) sur des côtés opposés de l'axe longitudinal (701) de la feuille de dessus (700), chaque revers de jambe joint à la feuille de dessus (700) ou à la feuille de fond (25), un premier revers de jambe (3401) et un deuxième revers de jambe (3402) ayant un premier bord terminal libre (6601) et un deuxième bord terminal libre (6602) dans une direction longitudinale, respectivement,

dans lequel la distance entre le premier bord terminal libre (6601) et le deuxième bord terminal libre (6602) n'est pas plus longue que la distance entre un bord interne de la première zone de liaison (719) et un bord interne de la deuxième zone de liaison (729) dans un état à plat.

17. Article absorbant (20) selon la revendication 16, dans lequel le premier substrat distinct (710) a des premier et deuxième bords latéraux internes (713, 714) chevauchant le deuxième substrat distinct (720), dans lequel la distance entre le premier bord terminal libre (6601) du premier revers de jambe (3401) et le deuxième bord terminal libre (6602) du deuxième revers de jambe (3402) est plus courte que la distance entre les premier et deuxième bords latéraux internes (713, 714) du premier substrat distinct (710).

18. Article absorbant (20) selon la revendication 16, dans lequel le deuxième substrat distinct (720) comprend un élément choisi dans le groupe constitué d'une pluralité de saillies, d'une pluralité d'évidements et d'une pluralité d'ouvertures et de combinaisons de ceux-ci.

19. Article absorbant (20) selon l'une quelconque des revendications 16 à 18, dans lequel la double zone a une distance de 8 à 20 mm.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13A

FIG. 13B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015250660 A
- US 2017056256 A
- US 2015250658 A
- WO 2015134371 A